Europäisches Patentamt

(19) **European Patent Office**

**Office européen des brevets**

(11) Publication number: **0 030 016**
**A2**

(12) # EUROPEAN PATENT APPLICATION

(21) Application number: **80107430.3**

(22) Date of filing: **27.11.80**

(51) Int. Cl.³: **C 12 N 5/00**
**//G01N33/54**

(30) Priority: **28.11.79 GB 7940980**

(43) Date of publication of application:
**10.06.81 Bulletin 81/23**

(84) Designated Contracting States:
**BE CH DE FR GB LI NL SE**

(71) Applicant: **THE WELLCOME FOUNDATION LIMITED**
**183-193 Euston Road**
**London NW1 2BP(GB)**

(72) Inventor: **Almeida, June Dalziel**
**72 The Hall Foxes Dale Blackheath**
**London S.E.3(GB)**

(72) Inventor: **Webster, Terence Roy**
**46 Greenways**
**Beckenham Kent(GB)**

(74) Representative: **Berg, Wilhelm, Dr. et al,**
**Dr. Berg, Dipl.-Ing. Stapf, Dipl.-Ing. Schwabe, Dr. Dr.**
**Sandmair Mauerkircherstrasse 45**
**D-8000 München 80(DE)**

(54) Method of preserving monolayers of tissue culture cells, and tissue culture cells so preserved.

(57) A method of preserving fixed monolayers of tissue culture cells grown on a solid, inert support which comprises coating the cells with a water insoluble wax. Also provided are tissue culture cells preserved by this method.

EP 0 030 016 A2

Attorney's file: 50 127                    27th November 1980

The Wellcome Foundation Ltd.
London / Great Britain

Improvements in and Relating to the Preservation of Monolayers
of Tissue Culture Cells

This invention relates to an improved method of preserving monolayers of tissue culture cells for use in immunofluorescent diagnostic techniques for the rapid detection of microbial infections, and in particular it relates to monolayers of tissue culture cells preserved by this method.

Immunofluroescent staining techniques are widely used in hospitals in diagnostic work, and in particular for the rapid detection of virus infections. One such technique, known as indirect immunofluorescence, involves growing the micro-organsim under study, for example mumps, measles, influenza, parainfluenza, herpes or adenovirus, in susceptible tissue culture cells on slides, fixing the cells, allowing a sample of test liquid suspected of containing antibodies to the specific virus to interact with the viral antigens in the cells, adding anti-species antibody, such as anti-human antibody, conjugated to a fluorescent dye such as fluoroscein - isothiocyanate or rhodamin and finally examining the cells under the fluorescent microscope.

The capacity of the antigens to react with the fluorescent labelled anti-species antibody, via the

GRHH/DD/A612 /October, 1980.

intermediary of the antigen specific antibody, decreases rapidly if the infected cultures are stored at room temperature. Thus it was found that storage of the fixed cells at $-20^{o}C$ or below would maintain the integrity of the cells and the ability of the antigen to react with antibody, and thereby ensuring a constant readily available supply of slides for diagnostic purposes. Such a storage method clearly suffers from the disadvantage that prepared slides must be transported in a frozen condition, as well as requiring clinical laboratories which use such preparations, to have at their disposal a freezer for storing them, thus the slides would occupy valuable freezer spece which could be used for other purposes.

Blaskovic and Rohoman described in <u>Medical Laboratory Sciences</u>, 1976, <u>34</u>, 175-176, a method for the fixation and storage of coverslips and microscope slides for immunofluorescent staining which used an aerosol histological fixative called Fisher 'Cyto Prep' Cytological Fixative (Fisher Scientific Company Limited, 711 Forbes Ave., Pittsburgh, Pa., 15219, U.S.A.). Cyto Prep is a 'spray on protection' for cytological smears. Blaskovic found that by spraying coverslips of fixed infected monolayers,for immunofluorescent work,with Cyto Prep, they could be stored at $4^{o}C$, the temperature of the average refrigerator,

GRHH/DD/A612 /October, 1980.

for periods of up to a year without showing adverse effects. These authors proposed to investigate the use of 'Smear-Fix' (Raymond A. Lamb, London NW10 6JL, England) for the same purpose, however, use of this aerosol fixative and storage at 4°C does not prevent the antigens from losing the capacity to interact · with fluorescent labelled antibodies.

It has now been found that by coating fixed monolayers of tissue culture cells, grown on a solid, inert support, with wax, such preparations can be stored at room temperature for periods in excess of 6 months, without any adverse effects.

Wax is commonly used in the preparation of tissues in microtomy as described in chapter 4 of 'Carleton's Histology Technique, 4th edition, published by Drury & Wallington, Oxford Press, 1967. In this technique fragments of tissue are impregnated with molten wax and subsequently allowed to solidify by cooling. Thus the function of the wax is to permeate the tissue and thereby produce a solid block from which thin sections may be cut for microscopical examination.

According to one aspect of the invention there is provided a method of preserving fixed monolayers of tissue culture cells grown on a solid, inert support, by coating the cells on the support with water insoluble

0030016

wax. Preferably the cells are coated with paraffin wax. Another suitable wax that may be used is beeswax.

The monolayers may be derived from several types of cell culture, and from several different species.

One type of cell culture used for propagating viruses comprises primary cells which are sensitive to growth of many viruses for example, influenza virus grows well in primary calf kidney. These cells may be prepared by standard techniques known in the art and described in such textbooks as "A Manual of Basic Virological Techniques" by Rovozzo. G. C., and Burke C.N., 1973, published by Prentice Hall. Alternatively established cell lines can be used for the propagation of micro-organisms, for example the human diploid MRC-5 cell line or Vero cells. The cells selected will thus depend on the micro-organism to be propagated, i.e. in what type and species of cell the organism grows best, as documented in the literature.

The preferred type of solid and inert support upon which the monolayers are grown is the microscope slide, which may be made of glass, plastic or any other biologically inert, solid, transparent material. Alternatively coverslips may be used or any other support suitable for examination purposes under the fluorescent microscope.

GRHH/DD/A612 /October, 1980.

The tissue culture monolayers must be fixed so that the cells will be preserved in the best condition possible. For this purpose any histological fixative may be used for example formaldehyde, glutaraldehyde or an alcohol such as methanol or ethanol, however, for fluorescent studies the most preferred fixative is acetone. Such fixatives combine the properties of preserving the integrity of the cells, whilst partially denaturing the surface protein with the result that the cells are immobilised and 'stick' to the support. After fixation by acetone the cells are allowed to dry in air.

The fixed, dried tissue culture monolayers on the support may be coated in a water insoluble wax by merely dipping or immersing them in molten wax, such as paraffin wax. Paraffin wax is usally classified according to its melting or congealing point, thus wax with a melting point of $45^{\circ}C$ to $50^{\circ}C$ is soft, whilst waxes which melt at $55^{\circ}C$ to $60^{\circ}C$ are correspondingly harder in consistency. The choice of wax used may depend on the average temperature of the storage area.

Such uninfected monolayers preserved by the method of the invention may be used as controls in the indirect immunofluorescent technique, however for this method monolayers infected with a known micro-organism are also required, and can also be preserved by the method of the invention. Infected monolayers preserved by this method may also be used as controls in direct immunofluorescent examinations.   The monolayers may be infected with such micro-organisms as viruses, protozoa, chlamydia, bacteria or rickettsiae which will grow in tissue culture.

One procedure for preparing and preserving slides for immunofluorescent examination comprises initially growing the cells selected, by incubating them in the presence of a suitable growth medium, in a tissue culture flask.   When the cells have formed a confluent layer on the surface of the flask they are inoculated with the selected micro-organism, for example a virus such as measles virus, and incubated for a further period of time which can vary from 1 to 10 days. As soon as the infected cells start to show a cytopathic effect (C.P.E.), or in the case of micro-organisms which do not produce a C.P.E., after a period of time when it can be considered that a proportion of the cells would be infected by that micro-organsim, the cells are removed from the flask by using for example trypsin and/or versene.   The cells are then resuspended in fresh medium and seeded onto a sterile support such as a microscope slide.   The slides are then incubated for between 6 and 18 hours until they have grown to produce a monolayer of cells on the slide surface. The slides are then removed from the incubator, washed with, for example, phosphate buffered saline and then transferred to 100% cold acetone, where they remain for at least 10 minutes. in order to fix the cells. The slides are then removed from the acetone and air dried.

The fixed slides are then immersed in molten paraffin wax (congealing point 54$^{o}$C) until the wax becomes clear again indicating that the slide and cells have equilibriated with the temperature of the wax. This may take as little as two seconds, or as long as 10 seconds. The slides are then removed from the wax, allowed to drain and left for the wax to set.

Slides prepared by this method may be stored at room temperature, i.e. about 25$^{o}$C, and can be stored at any temperature up to and including 37$^{o}$C without affecting them adversely.

In a second aspect of the invention there is provided tissue culture cells fixed on a solid, inert support and coated with water insoluble wax. The fixed tissue culture cells may or may not be infected with a micro-organism such as a virus, protozoa or chlamydia.

When such wax coated slides are required for diagnostic purposes, the slides are immersed in a solvent suitable for dissolving the wax, e.g. 100% xylene, and stirred for up to five minutes, before being removed and transferred to a solvent suitable for allowing slides to be either air dried or transferred to an aqueous solution, i.e. acetone or alcohol. The slides once air dried are ready for staining.

For the indirect immunofluorescent technique, a sample of blood is taken from a patient suspected of suffering, for example from a measles virus infection, and allowed to clot. The serum is then removed and added to one or more slides of fixed tissue culture monolayers infected with measles virus and allowed to react. The slides are then usually washed twice in a suitable buffer such as phosphate buffered saline Then, if the patient is a human, anti-human antisera, which has been raised in for example a rabbit by repeated injections of human serum , and which has been conjugated with a fluorescent dye such as fluoroscien isothiocyan-ate is added to the slide and allowed to react for 20 to 40 minutes preferably 30 minutes. The slides may then be washed again in buffer, followed by distilled water and dried ready for examination under the fluorescent microscope. For the indirect method it is usual to have a control slide comprising an uninfected monolayer which is otherwise treated and stained in exactly the same manner as an infected monolayer. This is required in order to eliminate any inadvertant and non-specific reaction between the patient's serum and the tissue culture cells.

The stained slides are then examined under the fluorescent microscope where any interaction between the patient's serum antibodies and the virus infected cells will be manifested by discrete fluorescent dots. Where the monolayer is heavily infected with virus and the patient has a high titre of antibody against measles, indicating a recent or current infection, the whole slide may appear as a solid fluorescent mass. Following comparison with the control slide fluorescence is scored on a scale from - to ++++.

The direct immunofluroescent technique, can be used for detection of micro-organisms and for this a sample of body fluid, e.g. blood, saliva, urine, cerebrospinal fluid etc., is taken from a patient suspected of suffering, for example from an influenza A infection, and added to a slide covered with a fresh monolayer of tissue culture cells, which influenza A virus will infect and multiply in, and allowed to react. The slide is then washed in a buffer and incubated for from 6 to 18 hours, after which time they are removed from the incubator, washed in a buffer and fixed by immersion in, for example, acetone for at least 10 minutes. The slides are then removed from the acetone and air dried.

Anti influenza A anti-serum, which has been raised in a laboratory animal, e.g. rabbit, by repeated injections of influenza A virus, and which has been conjugated with a fluorescent dye suitable for fluorescent microscopy such as fluorosceinisothiocyanate, is added to the slide and allowed to react for 20 to 40 minutes preferably 30 minutes. This anti-serum is also added to a control slide comprising a monolayer of fixed tissue culture cells infected with influenza A virus, and which has been preserved by the method of the present invention. Following washing in buffer and distilled water both slides are examined under the fluorescent microscope. When there is observable fluorescence on the slide treated with the patient's body fluid, and also on the control slide provided as above, then it can be presumed that the patient was suffering from an influenza A infection.

For use in the direct immunofluorescent diagnostic test the tissue culture cells fixed on a solid, inert support and coated with a water insoluble wax, and infected with a known micro-organism, e.g. influenza A virus, a closed vessel containing anti-serum having influenza A antibodies, together with instructions for use of them in the direct test, as set out above, may be presented and packaged in a box or container, thereby providing a diagnostic kit.

According to a further aspect of the invention there is provided a diagnostic kit, for use in the direct immunofluorescent technique comprising tissue culture cells fixed on a solid, inert support and coated with a water insoluble wax, and infected with a known micro-organism, a vessel containing anti-serum having specific antibodies against the aforementioned micro-organism and conjugated to a fluorescent dye, together with instructions for use as hereinbefore specified.

Coating slide cultures of cells, whether infected with a micro-organism or not, with a water insoluble wax provides a simple, cheap and safe method of preserving the cells and the antigen they may contain, and also has the advantage that the slides can be stored at room temperature without impairing the ability of antigens in the cells to interact with antibodies.

The present invention will be further illustrated by way of reference to the following examples, which are not intended to limit the invention in any way.

Example 1

Hep$_2$ cells (human epidermoid carcinoma of the larynx, ATCC No. CCL 23) were incubated in two tissue culture flasks in the presence of Eagles MEM (minimum essential medium) growth medium supplemented with amino acids, vitamins, 10% foetal calf serum, penicillin, streptomycin and 0.1% sodium bicarbonate buffer, at 37°C until they had become confluent. Once the cultures had become confluent the growth medium was removed from one flask and replaced by Eagles MEM maintenance medium containing 1-2% foetal calf serum and 0.2% sodium bicarbonate; this culture was designated the control (normal, uninfected) culture. The growth medium was drained from the remaining flask culture, and the culture was inoculated with 1 ml of a preparation of Herpes simplex type 1 virus. The virus was allowed to adsorb for 1 hour at $37^{o}C$ after which the culture was refed with the same maintenance medium as was used for the control. The control and infected cultures were then incubated at $37^{o}C$ until a cytopathic effect was seen in the infected culture when examined under the microscope. The maintenance medium was removed from both the control and infected cultures, and each cell sheet was overlaid with 10 ml prewarmed trypsinisation medium (0.025% trypsin in 0.05% versene) and left for 1 minute at room temperature. The trypsinisation medium was poured off until it began to drip, thus leaving from 0.5 to 1 ml in each flask. The flasks were left on the bench for 1 minute. The trypsinised cells were removed from both flasks by pipette and the concentration of them was adjusted using growth medium so as not to over seed the slides, using a calibrated pipette. The cells were seeded onto glass slides which had been previously washed, and sterilized using UV light.  The seeded slides were then incubated at $37^{o}$C in a closed container, which is humidified to prevent the medium on the slides evaporating, until the cells became confluent.  When the cells had grown sufficiently as determined by microscopical examination, the slides were removed from the incubator and washed in phosphate buffered saline for one minute with stirring. They were then immersed in cold acetone to fix the cells and after 10 minutes the slides were removed from the acetone and air dried.

A quarter of the number of prepared slides of infected tissue culture and a quarter of the number of prepared slides of control (uninfected) culture were immersed in molten paraffin wax (congealing point $54^{o}$C obtained from BDH, Poole, Dorset, England) for approximately 5 seconds, at which time the slides had equilibrated with the temperature of the wax as denoted by the initial wax deposit on the slide becoming clear. The slides were then removed from the wax bath, drained and the wax was allowed to set.  The slides were then placed at room temperature for storage.

A quarter of the number of prepared slides of infected tissue culture was placed at each of the following temperatures, as were slides of uninfected tissue cultures:-  $-20^{o}$C, $37^{o}$C and room temperature.

These slides were storage temperature controls.

After eight weeks all the slides were taken from their storage areas. The infected and uninfected slide cultures which had been coated with paraffin wax were immersed in neat xylene for 5 minutes, then transferred to neat acetone for 10 minutes and then dried in air.

## Staining

All slides were stained by the indirect fluorescent technique, which involved immersing them in sheep antiherpes serum (dilution 1/100, 1/1000 and 1/10000) for 30 minutes at $37^{o}C$ in a humidified container, after which they were removed and washed twice with phosphate buffered saline. The slides were then dipped in a 1/40 dilution of rabbit anti-sheep serum which had been conjugated to fluoroscein isothiocyanate, and then washed again twice in phosphate buffered saline. The slides were finally washed in distilled water and dried. A coverslip was fixed on each slide using the histological mountant Histomount (Registered Trade Mark) obtained from Hughes and Hughes Ltd., Romford, Essex, the slides were then examined under a fluorescent microscope using a UV light source.

Results                    Relative Fluorescence after
                           eight weeks storage

| Slide | Dilution of sheep anti-herpes serum | | |
|---|---|---|---|
| | 1/100 | 1/1000 | 1/10000 |
| Infected slide, wax coated, room temperature storage | 3-4+ | 1-2+ | trace |
| (good contrast at all dilutions) | | | |
| Control slide, wax coated, room temperature storage | -Ve | -Ve | -Ve |
| Infected slide, uncoated, -20° storage | 4+ | 3+ | 1+ |
| (good contrast at all dilutions) | | | |
| Control slide, uncoated, -20°C storage | -Ve | -Ve | -Ve |
| Infected slide, uncoated, 37°C storage | 3+ | 2+ | trace |
| (poor contrast) | | | |
| Control slide, uncoated, 37°C storage | -Ve | -Ve | -Ve |
| Infected slide, room temperature storage, uncoated | 2+ | 1+ | ± |
| (poor contrast) | | | |
| Control slide, room, temperature storage, uncoated | -Ve | -Ve | -Ve |

Calibration of results

4+ - very bright fluorescence to 1+ = very dim fluore-

scence;  -Ve = no fluorescence.

From these results it can be seen that after eight weeks storage tissue culture slides preserved by coating with paraffin wax exhibit a degree and quality of fluorescence almost equal to slides stored under traditional methods, i.e. $-20^{o}C$, and have the great advantage that such coated slides can be stored at room temperature.

Example 2

Tissue culture slides are prepared in a manner similar to that described in Example 1 except that primary calf kidney cells are used instead of $Hep_2$ cells, and cultures are infected with influenza A virus instead of herpes virus. All slides are stained by adding to the slides bovine serum containing anti-influenza A antibodies (dilutions 1/30, 1/60, 1/90, 1/120), obtainable from Dr. J. Almeida, The Wellcome Foundation Limited, Beckenham, England, followed by washing then the addition of rabbit anti-bovine serum conjugated to fluoroscein-isothiocyanate, obtainable from Wellcome Reagents, Beckenham, Kent, England. The slides are then processed as described in Example 1 and examined under the fluorescent microscope.

GRHH/DD/A612/October, 1980.

Results              Relative Fluorescence

| Slide | Dilution of bovine anti-influenza A | | | |
|-------|------|------|------|-------|
| | 1/30 | 1/60 | 1/90 | 1/120 |
| Infected slide, wax coated, room temperature storage | 4+ | 3+ | 2+ | 2+ |
| Control slide, wax coated, room temperature storage | -Ve | -Ve | -Ve | -Ve |
| Infected slide -20°C storage, uncoated | 4+ | 4+ | 3+ | 2+ |
| Control slide -20°C storage, uncoated | -Ve | -Ve | -Ve | -Ve |
| Infected slide 37°C storage, uncoated | 1+ | 1+ | $\pm$ | -Ve |
| Control slide 37°C storage uncoated | -Ve | -Ve | -Ve | -Ve |
| Infected slide, room temperature storage, uncoated | 2+ | 1+ | $\pm$ | -Ve |
| Control slide, room temperature storage, uncoated | -Ve | -Ve | -Ve | -Ve |

Claims

1.  A method of preserving fixed monolayers of tissue culture cells grown on a solid, inert support characterised in that the cells grown on the support are coated with a water insoluble wax.

2.  A method as claimed in claim 1 characterised in that the water insoluble wax is paraffin wax.

3.  A method as claimed in either claim 1 or claim 2 characterised in that the cells grown on the support are primary cells.

4.  A method as claimed in either claim 1 or claim 2 characterised in that the cells grown on the support are an established cell line.

5.  A method as claimed in any one of the preceding claims characterised in that the cells are infected with a micro-organism.

GHH/em/21.10.80

6. A method as claimed in any one of the preceding claims characterised in that the solid, inert support is a microscope slide or coverslip.

7. Tissue culture cells fixed on a solid, inert support characterised in that the cells are coated with a water insoluble wax.

8. Tissue culture cells as claimed in claim 7 characterised in that the water insoluble wax is paraffin wax.

9. Tissue culture cells as claimed in either claim 7 or claim 8 characterised in that the cells are primary cells.

10. Tissue culture cells as claimed in either claim 7 or claim 8 characterised in that the cells are an established cell line.

11. Tissue culture cells as claimed in any one of claims 7 to 10 characterised in that they are infected with a micro-organism.